# FASCICULE DE BREVET EUROPEEN

(11) **EP 2 482 762 B1**
(45) Date de publication et mention de la délivrance du brevet: **04.11.2015**
(21) Numéro de dépôt: 10845638.5
(22) Date de dépôt: 27.09.2010
(51) Int. Cl.: A61F 2/34, A61F 2/46

(54) **PROCEDE DE FABRICATION D'UN COTYLE PROTHETIQUE DE HANCHE**
VERFAHREN ZUR HERSTELLUNG EINER HÜFTGELENKPFANNENPROTHESE
METHOD FOR MANUFACTURING A PROSTHETIC HIP ACETABULUM

(30) Priorité: 28.09.2009 FR 0956715
(43) Date de publication de la demande: 08.08.2012
(73) Titulaire: Inventorio SA, 1196 Gland (CH)
(72) Inventeur: Gradel, Thomas, 74970 Marignier (FR)
(74) Mandataire: Poncet, Jean-François
(86) Numéro de dépôt international: PCT/IB2010/054341
(87) Numéro de publication internationale: WO 2011/098868

(56) Documents cités:
- FR-A1- 2 876 278
- FR-A1- 2 909 541
- FR-A1- 2 916 960
- US-A1- 2004 186 586

## Description

### DOMAINE TECHNIQUE DE L'INVENTION

La présente invention concerne un procédé de fabrication d'un cotyle prothétique destiné à remplacer le cotyle naturel de la hanche.

Une prothèse totale de hanche comprend deux parties constituant une articulation à rotule, à savoir une partie femelle destinée à remplacer le cotyle naturel de la hanche et une partie mâle destinée à remplacer la tête du fémur. La partie mâle de l'articulation comporte généralement une tige destinée à pénétrer dans le canal médullaire du fémur, et dont l'extrémité proximale se raccorde par un col à une tête sphérique destinée à pénétrer dans la partie femelle. La partie femelle de l'articulation, qui doit remplacer le cotyle naturel de la hanche, comprend habituellement une cupule d'insertion sensiblement hémisphérique, qui vient se loger dans une cavité cotyloïdienne préparée de l'os du bassin. De façon courante, la cupule d'insertion est métallique.

Lors de la pose de la cupule d'insertion dans la cavité cotyloïdienne, il faut pouvoir utiliser un impacteur qui permet de tenir et manipuler la cupule d'insertion et de lui appliquer une force d'enfoncement dans la cavité cotyloïdienne de l'os avec une bonne orientation, pendant une durée suffisante notamment pour la prise d'un ciment entre la surface externe de la cupule d'insertion et la cavité cotyloïdienne de l'os.

Le document FR 2 916 960 A1 décrit une cupule d'insertion à rainure circulaire externe formée parallèlement au plan équatorial de la cupule pour une préhension de la cupule par des mors articulés d'un impacteur.

Le document FR 2 909 541 A1 décrit une cupule d'insertion à face extérieure d'ancrage convexe et hémisphérique. Pour tenir cette cupule lors de son impaction, il est prévu une gorge annulaire externe au voisinage de son bord libre annulaire. Cette gorge annulaire permet la fixation d'un impacteur comportant un corps de base à au moins deux pattes élastiques en forme de crochet.

Dans ce document, la force de retenue de la cupule par l'impacteur est en corrélation avec et sensiblement égale à la force qu'il est nécessaire d'appliquer pour écarter les pattes élastiques. Ceci signifie que meilleure est la tenue de la cupule par l'impacteur plus l'écartement des pattes est difficile, ce qui oblige l'utilisateur à peiner d'autant plus pour fixer l'impacteur à la cupule.

Il y a un besoin de permettre une fixation aisée d'un impacteur à une cupule tout en procurant une tenue fiable de la cupule.

Le document WO 2006/040483 A1 décrit un cotyle prothétique de hanche comprenant une cupule d'insertion ayant :
- une face extérieure d'ancrage convexe sensiblement hémisphérique conformée pour être ancrée dans une cavité cotyloïdienne du bassin d'un patient,
- une face de réception interne concave à bord annulaire et à face d'ouverture comprise dans un plan d'ouverture,
- une structure annulaire de réception conformée de façon qu'un impacteur puisse être fixé à ladite structure annulaire de réception pour l'impaction de la cupule dans la cavité cotyloïdienne du bassin d'un patient.

Dans le document WO 2006/040483 A1, l'insert de pose et d'orientation est refroidi pour se contracter et permettre son introduction dans la cupule d'insertion, puis ramené à température ambiante pour se dilater et se solidariser fortement à la cupule d'insertion. Afin de diminuer suffisamment les dimensions de l'insert de pose et d'orientation, celui-ci est refroidi dans une enceinte réfrigérée. On a parfois constaté la présence de liquide à l'intérieur de l'enceinte réfrigérée, provenant d'un phénomène de condensation. Des liquides de condensats contaminés par des bactéries ou des microbes peuvent ainsi se former et/ou se déposer sur l'insert de pose et d'orientation, qui contamine ensuite la cupule d'insertion après assemblage de l'insert de pose et d'orientation et de la cupule d'insertion. Ceci est particulièrement critique lorsque l'insert de pose et d'orientation est en matière plastique : les matières plastiques utilisables pour l'insert de pose et d'orientation, comme le polyéthylène par exemple, ont tendance à absorber les liquides. Il y a donc un risque que les inserts de pose et d'orientation en matière plastique absorbent des liquides de condensats contaminés par des bactéries ou des microbes, ce qui entraîne alors une contamination en profondeur des inserts de pose et d'orientation.

Et il est en outre utile d'assurer entre la cupule et l'impacteur une liaison encore plus forte que ce qui est rendu possible par les enseignements du document WO 2006/040483 A1.

### EXPOSE DE L'INVENTION

Un problème proposé par l'invention est de proposer un procédé de fabrication d'un cotyle prothétique de hanche à cupule d'insertion qui puisse être fixé de façon encore plus fiable et robuste à un impacteur, en limitant les efforts à accomplir et en évitant tout risque de contamination.

Pour atteindre ces buts, ainsi que d'autres, l'invention propose un procédé de fabrication comprenant les étapes de :
a) prévoir une cupule d'insertion ayant une face de réception interne concave à face d'ouverture comprise dans un plan d'ouverture, et ayant une structure annulaire de réception externe,
b) prévoir un insert de pose et d'orientation à structure périphérique annulaire de fixation conformée pour coopérer avec la face de réception de la structure annulaire externe de réception en s'engageant autour de la structure annulaire externe de réception,
c) chauffer l'insert de pose et d'orientation afin d'en augmenter les dimensions,
d) engager la structure périphérique annulaire de fixation autour de la face de réception de la structure annulaire externe de réception,
e) ramener à température ambiante l'insert de pose et d'orientation pour en diminuer les dimensions de façon à obtenir un serrage radial de la structure périphérique annulaire de fixation sur la structure annulaire externe de réception.

Un tel procédé de fabrication permet d'obtenir un serrage radial satisfaisant de l'insert de pose et d'orientation sur la cupule d'insertion.

L'engagement de l'insert de pose et d'orientation se fait sans trop d'efforts autour de la structure annulaire externe de réception, du fait de la dilatation de l'insert de pose et d'orientation par chauffage.

Mais la séparation de la cupule et de l'insert de pose et d'orientation nécessite des efforts importants après refroidissement, ce qui permet d'impacter et orienter la cupule de manière satisfaisante.

La retenue en force par un serrage radial de l'insert de pose et d'orientation sur la cupule d'insertion assure une fixation fiable et solide qui permet d'endurer les efforts subis lors de l'impaction de la cupule d'insertion. Le serrage radial de la structure périphérique annulaire de fixation sur la structure annulaire externe de réception permet d'assurer cette fiabilité de fixation sans compliquer outre mesure la forme de la structure annulaire externe de réception.

La retenue de l'insert de pose et d'orientation sur la cupule d'insertion est plus fiable que celle du document WO 2006/040483 A1, notamment grâce au fait que le serrage radial se fait sur une structure annulaire de réception qui est externe et qui présente donc une plus grande surface de contact avec l'insert de pose et d'orientation.

Enfin, le serrage radial de la structure périphérique annulaire de fixation sur la structure annulaire externe de réception induit un état de précontrainte dans la cupule d'insertion, et cette précontrainte limite les risques de déformation de la cupule d'insertion lors de son impaction dans le cotyle du bassin d'un patient. En effet, lorsque la cupule d'insertion présente une épaisseur relativement faible au voisinage de son bord annulaire supérieur, par exemple inférieur à 3 mm, il se produit parfois des phénomènes d'ovalisation de la cupule d'insertion (si celle-ci est en un matériau déformable tel que du métal ou du plastique par exemple) qui rendent ensuite impossible la pénétration de la partie mâle de l'articulation prothétique dans la cupule d'insertion, ou qui génèrent des contraintes mécaniques internes inégalement réparties susceptibles de conduire au bris de la cupule d'insertion (si celle-ci est en un matériau fragile tel que de la céramique par exemple), ou qui génèrent des contraintes mécaniques internes inégalement réparties susceptibles de dégrader ultérieurement l'insert articulaire, notamment si celui-ci est en céramique.

Dans le procédé de fabrication de la présente invention, le fait de prévoir une cupule d'insertion à structure annulaire de réception externe sur laquelle vient se serrer un insert de pose et d'orientation par retour à température ambiante après dilatation par chauffage permet d'éviter le problème de contamination des inserts de pose et d'orientation, notamment lorsque ceux-ci sont en polyéthylène.

Dans le cadre de la présente invention, la face de réception interne concave peut être une face de glissement pour la réception d'un insert articulaire mobile par rapport à la cupule d'insertion (pour un cotyle à double mobilité par exemple) ou peut être conformée pour la réception d'un insert articulaire immobile par rapport à la cupule d'insertion (pour un cotyle à simple mobilité par exemple).

Avantageusement, le procédé peut comprendre, avant fixation de l'insert de pose et d'orientation sur la cupule d'insertion, l'étape supplémentaire b1) d'insérer un insert articulaire dans la face de réception interne concave de la cupule d'insertion.

La cupule d'insertion, l'insert articulaire et l'insert de pose et d'orientation peuvent ainsi être assemblés préalablement à l'usine, de sorte que le chirurgien n'a plus qu'à impacter l'ensemble ainsi formé sans avoir à se préoccuper de l'insert articulaire. En outre, l'insert articulaire peut alors être positionné correctement dans la cupule d'insertion sans que le chirurgien n'ait à intervenir. On limite ainsi les risques de mauvais positionnement de l'insert articulaire dans la cupule d'insertion qui peut conduire à une casse prématurée de l'insert articulaire.

Avantageusement, on peut prévoir que :
- l'insert articulaire est en céramique,
- l'insert de pose et d'orientation comporte des moyens élastiques de maintien de l'insert articulaire dans la cupule d'insertion,
- lorsque l'insert de pose et d'orientation est fixé à la cupule d'insertion, l'insert de pose et d'orientation ne vient en contact avec l'insert articulaire que selon les moyens élastiques de maintien de l'insert articulaire dans la cupule d'insertion.

Les moyens élastiques de maintien gardent l'insert articulaire correctement positionné dans la cupule d'insertion lors du transport, du stockage et des manipulations du chirurgien. Lorsque le chirurgien appliquera une force d'impaction sur la cupule d'insertion par l'intermédiaire de l'insert de pose et d'orientation, aucun choc ne sera transmis à l'insert articulaire en céramique puisque les moyens élastiques de maintien se déformeront. Il n'y a donc pas de risque pour le praticien de casser l'insert articulaire en céramique lors de l'impaction.

Avantageusement, le procédé peut en outre comporter une étape f) au cours de laquelle on stérilise l'ensemble ainsi formé et emballé dans une enveloppe de protection microbienne.

De préférence, on peut prévoir que l'insert articulaire est en céramique et que l'étape f) de stérilisation est réalisée par bombardement de rayons gamma, avantageusement selon une dose comprise entre environ 25 kGy et environ 40 kGy.

Dans le document WO 2006/040483 A1, la structure annulaire de réception comprend une surface annulaire de retenue, cylindrique ou légèrement conique, prolongeant la surface de la face de réception interne concave.

Ce prolongement sensiblement cylindrique vient cependant limiter le débattement angulaire possible de la tige fémorale prothétique. En outre, le prolongement cylindrique augmente le risque que la tige fémorale prothétique, qui est généralement en métal, vienne au contact de la cupule d'insertion. Lors d'un tel contact, si la cupule d'insertion est métallique, il se produit un phénomène de métallose par dégradation du bord de la cupule d'insertion et/ou de la tige fémorale prothétique. Cette dégradation génère des débris métalliques qui peuvent imprégner les tissus alentour et/ou endommager la prothèse. Et lorsque la cupule n'est pas métallique, et qu'elle est notamment en céramique ou en matière plastique telle que du PEEK par exemple, il y a un risque de bris partiel ou total de la cupule d'insertion.

C'est pourquoi la structure annulaire de réception peut de préférence être externe et située en retrait du plan d'ouverture.

Lorsque la structure annulaire de réception pour la fixation de l'impacteur est située en retrait du plan d'ouverture, en direction du sommet de la face extérieure d'ancrage convexe sensiblement hémisphérique, cette structure annulaire de réception ne limite plus le débattement angulaire de la tige fémorale prothétique qui sera installée. Simultanément, on limite le risque de contact entre la tige fémorale prothétique et le bord de la cupule d'insertion, ce qui réduit ainsi le risque de métallose et/ou de bris.

De préférence, l'insert de pose et d'orientation peut comporter une structure d'assemblage sur laquelle un impacteur peut être fixé de façon amovible.

Il est ainsi possible de monter en usine l'insert de pose et d'orientation sur la cupule d'insertion, de sorte que le chirurgien n'a plus qu'à fixer l'impacteur sur l'insert de pose et d'orientation pour procéder à l'impaction de la cupule d'insertion. Il n'y a pas de risque que le chirurgien vienne dégrader la face de réception interne concave de la cupule d'insertion, celle-ci étant protégée par l'insert de pose et d'orientation.

Avantageusement, on peut prévoir que :
- la structure annulaire externe de réception comprend un décrochement radial périphérique continu ou discontinu du bord annulaire, avec une face de réception orientée vers l'extérieur,
- l'insert de pose et d'orientation comporte une structure périphérique annulaire de fixation continue ou discontinue à face de liaison orientée vers l'intérieur, conformée pour coopérer avec la face de réception de la structure annulaire externe de réception.

Selon une première variante, on peut prévoir que :
- la face de réception de la structure annulaire externe de réception comporte une gorge de verrouillage périphérique discontinue,
- la face de liaison de la structure périphérique annulaire de fixation de l'insert de pose et d'orientation comporte une pluralité de nervures de verrouillage réparties en périphérie et conformées pour s'engager dans la gorge de verrouillage périphérique discontinue.

Selon une seconde variante, on peut prévoir que :
- l'une de la face de réception de la structure annulaire externe de réception ou de la face de liaison de la structure périphérique annulaire de fixation comporte une gorge de verrouillage annulaire périphérique continue,
- l'autre de la face de liaison de la structure périphérique annulaire de fixation ou de la face de réception de la structure annulaire externe de réception comporte une nervure de verrouillage annulaire périphérique continue ou discontinue conformée pour s'engager dans la gorge de verrouillage annulaire périphérique continue.

La gorge de verrouillage et la ou les nervures de verrouillage participent à la retenue de l'insert de pose et d'orientation sur la cupule d'insertion. On augmente également de façon importante la résistance de la liaison entre l'insert de pose et d'orientation et la cupule d'insertion pour endurer les couples d'orientation que le chirurgien transmet au moyen de l'impacteur.

Avantageusement, on peut prévoir que :
- le décrochement radial périphérique du bord annulaire a une épaisseur comprise entre environ 0,7 mm et environ 1,2 mm,
- ladite gorge de verrouillage périphérique discontinue ou ladite gorge de verrouillage annulaire périphérique continue de la structure annulaire externe de réception a une épaisseur radiale comprise entre environ 0,2 mm et environ 0,6 mm.

De telles dimensions sont compatibles avec une cupule d'insertion de faible épaisseur au voisinage de son bord annulaire supérieur, par exemple inférieure à 3 mm. L'utilisation d'une cupule d'insertion de faible épaisseur au voisinage de son bord annulaire supérieur permet d'augmenter le diamètre de la tête sphérique de la partie mâle de l'articulation et donc de limiter le risque de luxation.

De préférence, on peut prévoir que le décrochement radial périphérique du bord annulaire a une hauteur comprise entre environ 1 mm et environ 4 mm.

Une telle hauteur du décrochement radial périphérique procure une -surface de contact suffisante entre l'insert de pose et d'orientation et la cupule d'insertion pour le serrage radial, sans pour autant trop diminuer la surface de la face extérieure d'ancrage convexe destinée à venir en contact avec l'os. Ceci est d'autant plus important que la partie de face extérieure d'ancrage située sensiblement au voisinage du plan équatorial de celle-ci est une partie qui participe de façon importante à la retenue de la cupule d'insertion dans la cavité cotyloïdienne du bassin.

Avantageusement, on peut prévoir que ladite gorge de verrouillage périphérique discontinue ou ladite gorge de verrouillage annulaire périphérique continue de la structure annulaire externe de réception a une hauteur comprise entre environ 0,4 mm et environ 3 mm.

De préférence, on peut prévoir que l'insert de pose et d'orientation et la structure annulaire externe de réception de la cupule d'insertion sont conformés de telle sorte que, lorsque l'insert de pose et d'orientation est fixé à la structure annulaire externe de réception de la cupule d'insertion, l'insert de pose et d'orientation ne dépasse pas à l'extérieur d'une surface sensiblement hémisphérique définie par la face extérieure d'ancrage convexe sensiblement hémisphérique de la cupule d'insertion. On évite ainsi tout risque de conflit de l'insert de pose et d'orientation avec la masse osseuse présente au voisinage de la cavité cotyloïdienne préparée du bassin du patient.

Avantageusement, la structure périphérique annulaire de fixation de l'insert de pose et d'orientation peut présenter une épaisseur radiale sensiblement égale ou inférieure à l'épaisseur du décrochement radial du bord annulaire.

De préférence, l'insert de pose et d'orientation peut être en polyéthylène. Le polyéthylène est d'usage courant dans le domaine médical, peu onéreux et facile à usiner. Le polyéthylène ne risque en outre pas d'endommager une cupule d'insertion métallique, en céramique, en PEEK ou en un matériau plus dur que le polyéthylène.

De préférence, la structure d'assemblage peut comprendre un trou de fixation à taraudage interne pratiqué dans l'insert de pose et d'orientation, permettant le vissage d'une portion filetée correspondante de l'impacteur.

Avantageusement, le trou de fixation peut être un trou débouchant, apte à coopérer avec un outil de désolidarisation comportant une tige filetée apte à se visser dans le trou débouchant et qui a une extrémité distale conformée pour prendre appui, directement ou indirectement, contre la face de réception interne concave de la cupule d'insertion lors du vissage de la tige filetée dans le trou débouchant.

De préférence, on peut prévoir que :
- l'insert de pose et d'orientation est conformé de façon à ce qu'il reste un espace libre entre l'insert de pose et d'orientation et le fond de la face de réception interne concave, ou, le cas échéant, entre l'insert de pose et d'orientation et l'insert articulaire, une fois l'insert de pose et d'orientation fixé à la structure annulaire externe de réception de la cupule d'insertion,
- l'insert de pose et d'orientation est en contact de façon étanche selon sa face de liaison contre la face de réception de la structure annulaire externe de réception,
- le trou de fixation est un trou débouchant, mettant en communication avec l'extérieur l'espace libre entre l'insert de pose et d'orientation et le fond de la face de réception interne concave, ou, le cas échéant, entre l'insert de pose et d'orientation et l'insert articulaire, et dimensionné pour y engager l'extrémité d'une seringue de façon étanche.

### DESCRIPTION SOMMAIRE DES DESSINS

D'autres objets, caractéristiques et avantages de la présente invention ressortiront de la description suivante de variantes particulières, faite en relation avec les figures jointes, parmi lesquelles :
- la figure 4 est une vue en perspective d'une cupule d'insertion d'un cotyle prothétique selon une première variante de l'invention ;
- la figure 5 est une vue de côté de la cupule de la figure 4 ;
- la figure 6 est une vue en perspective d'un insert de pose et d'orientation destiné à être associé avec la cupule d'insertion des figures 4 et 5 ;
- la figure 7 est une vue en coupe de l'insert de pose et d'orientation de la figure 6 ;
- la figure 8 est une vue en perspective d'un insert de pose et d'orientation et d'une cupule d'insertion d'un cotyle prothétique de hanche selon une deuxième variante de l'invention ;
- la figure 9 est une vue en coupe de l'insert de pose et d'orientation de la figure 8 ;
- la figure 10 est une vue partielle en coupe de la cupule d'insertion de la figure 8 ;
- la figure 11 est une vue partielle en coupe de l'insert de pose et d'orientation de la figure 9 ;
- la figure 12 est une vue partielle en coupe de la cupule d'insertion et de l'insert de pose et d'orientation des figures 10 et 11 ;
- la figure 13 est une vue en perspective de la cupule d'insertion et de l'insert de pose et d'orientation de la figure 8 avec un insert articulaire immobile ;
- la figure 14 est une vue en coupe des éléments de la figure 13 une fois assemblés ; et
- la figure 15 est une vue en coupe d'une cupule d'insertion et d'un insert de pose et d'orientation une fois assemblés.

Certaines des figures jointes illustrent un objet qui ne fait pas partie de la présente invention mais qui est décrit ci-après et qui pourra faire l'objet d'une protection indépendante. Ces figures sont les suivantes :

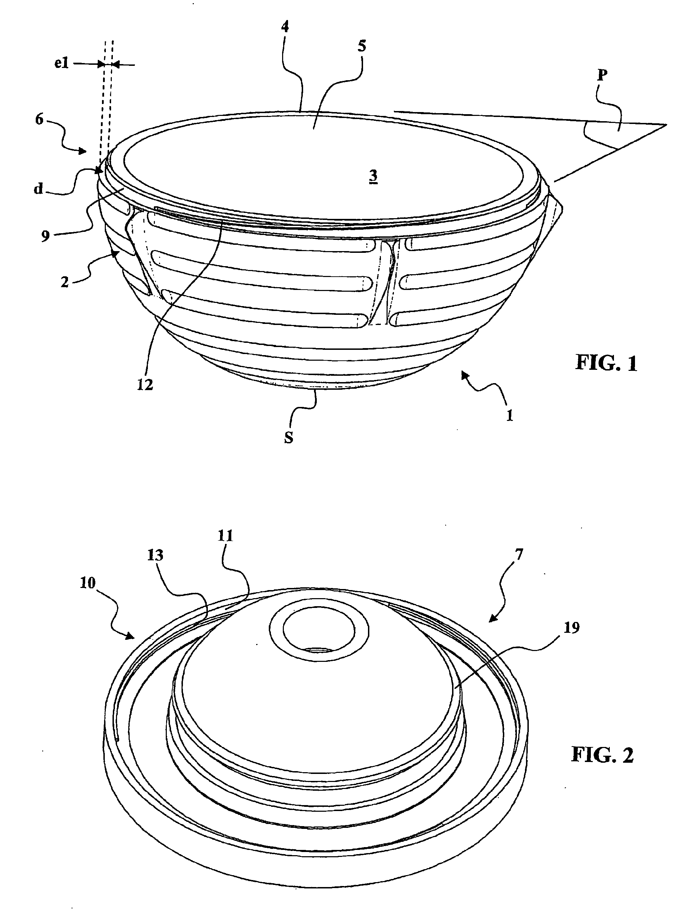

- la figure 1 qui est une vue en perspective d'une cupule d'insertion d'un cotyle prothétique ;
- la figure 2 qui est une vue en perspective d'un insert de pose et d'orientation destiné à être associé à la cupule d'insertion de la figure 1 ; et

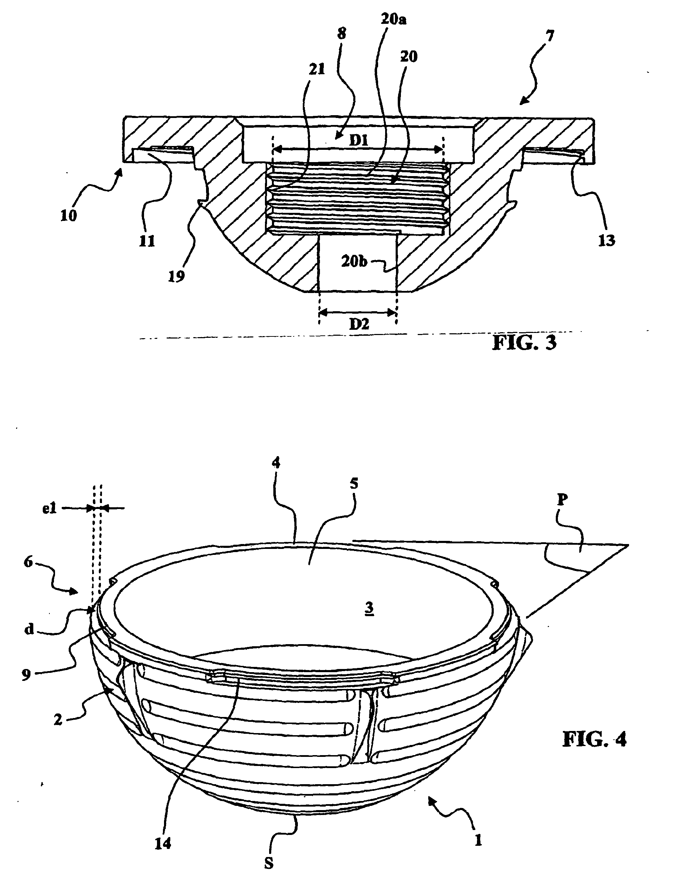

- la figure 3 qui est une vue en coupe de l'insert de pose et d'orientation de la figure 2.

### DESCRIPTION DES MODES DE REALISATION PREFERES

Sur chacune des figures 1, 4, 5 et 8 est représentée une cupule d'insertion 1 de cotyle prothétique de hanche. Cette cupule d'insertion 1 comprend :
- une face extérieure d'ancrage 2 convexe sensiblement hémisphérique conformée pour être ancrée dans une cavité cotyloïdienne préparée du bassin d'un patient, et une face de réception interne 3 concave à bord annulaire 4 et à face d'ouverture 5 comprise dans un plan d'ouverture P,
- une structure annulaire de réception 6 conformée de façon qu'un impacteur puisse être fixé à ladite structure annulaire de réception 6 pour l'impaction de la cupule d'insertion 1 dans la cavité cotyloïdienne du bassin d'un patient.

La structure annulaire de réception 6 est externe et située en retrait du plan d'ouverture P. En d'autres termes, la structure annulaire de réception 6 est décalée par rapport au plan d'ouverture P en direction du sommet S de la face extérieure d'ancrage 2.

La structure annulaire de réception 6 ne se situe ainsi pas en prolongement de la face de réception interne 3 en prolongeant celle-ci à l'écart du sommet S. On évite ainsi de limiter le débattement angulaire de la tige fémorale prothétique, et on réduit le risque de métallose et/ou de bris.

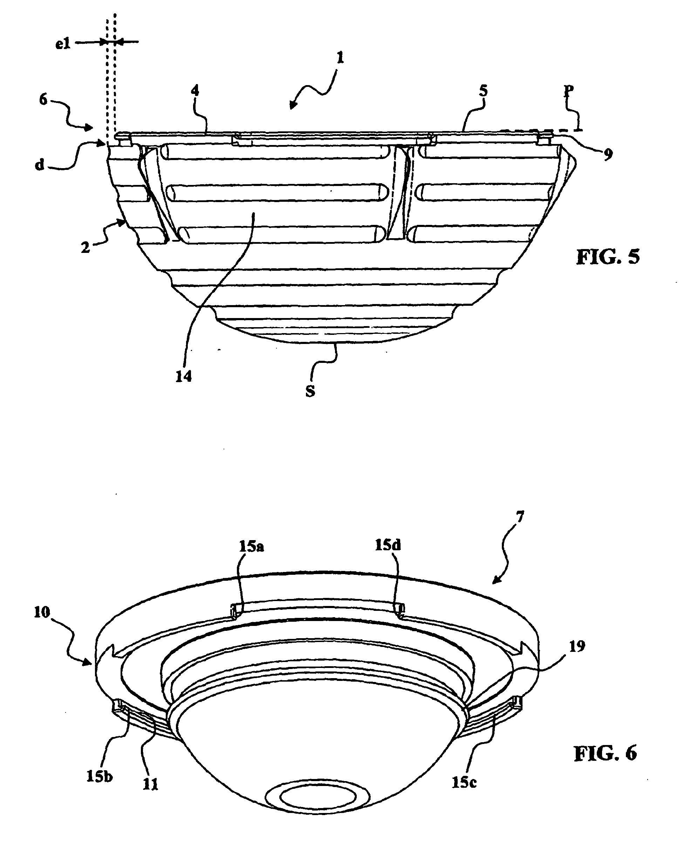

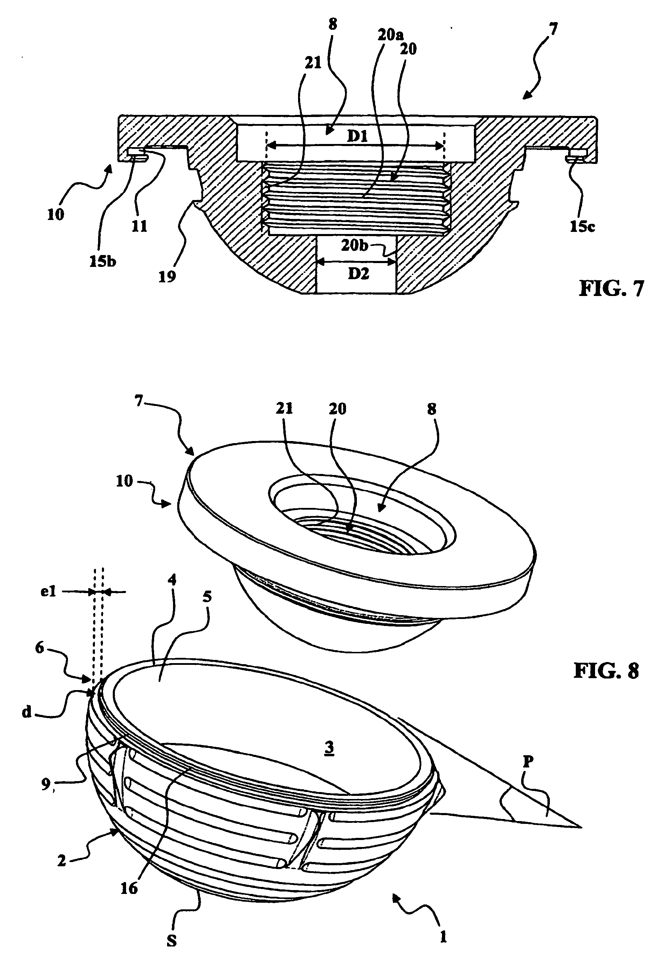

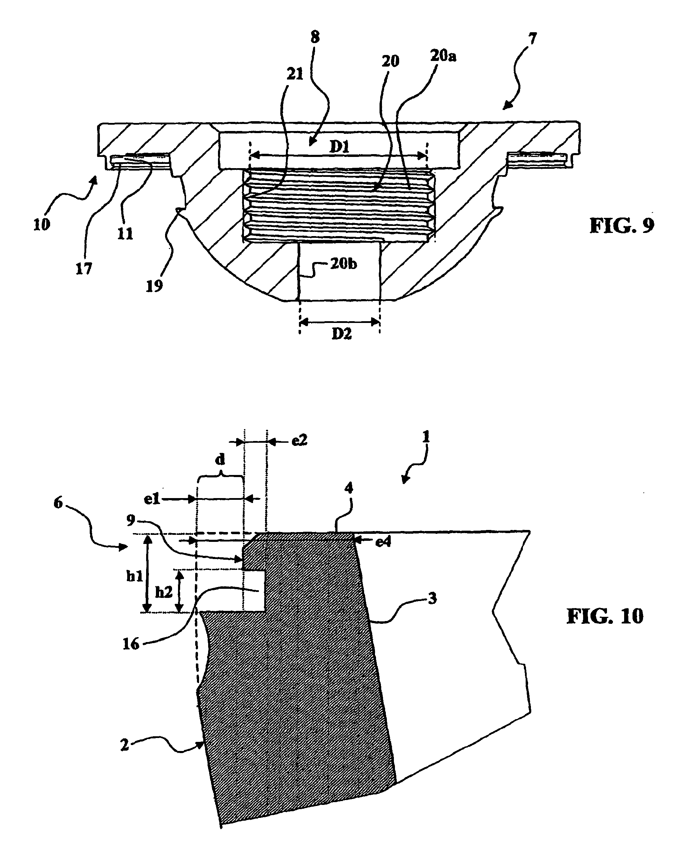

Sur les figures 2, 3, 6, 7 et 9 est représenté un insert de pose et d'orientation 7 destiné à être associé avec une cupule d'insertion 1. L'insert de pose et d'orientation 7 des figures 2 et 3 est destiné à être associé à la cupule d'insertion 1 de la figure 1. L'insert de pose et d'orientation 7 des figures 6 et 7 est destiné à être associé avec la cupule d'insertion 1 des figures 4, 5 et 8. L'insert de pose et d'orientation 7 de la figure 9 est destiné à être associé avec la cupule d'insertion 1 de la figure 8.

Chaque insert de pose et d'orientation 7 peut être fixé de façon amovible à la structure annulaire externe de réception 6 de la cupule d'insertion 1 et comporte une structure d'assemblage 8 sur laquelle un impacteur peut être fixé de façon amovible.

Sur les figures 1, 5 et 8, on voit que la structure annulaire externe de réception 6 comprend un décrochement radial périphérique d continu ou discontinu du bord annulaire 4, avec une face de réception 9 orientée vers l'extérieur. On voit sur les figures 2, 3, 6, 7 et 9 que chaque insert de pose et d'orientation 7 comporte une structure périphérique annulaire de fixation 10 continue ou discontinue à face de liaison 11 orientée vers l'intérieur, conformée pour coopérer avec la face de réception 9 de la structure annulaire externe de réception 6.

Dans l'objet des figures 1 à 3, la face de réception 9 de la structure annulaire externe de réception 6 comporte un filetage externe 12 tandis que la face de liaison 11 de la structure périphérique annulaire de fixation 10 de l'insert de pose et d'orientation 7 comporte un filetage interne 13 conformé pour coopérer par vissage avec le filetage externe 12.

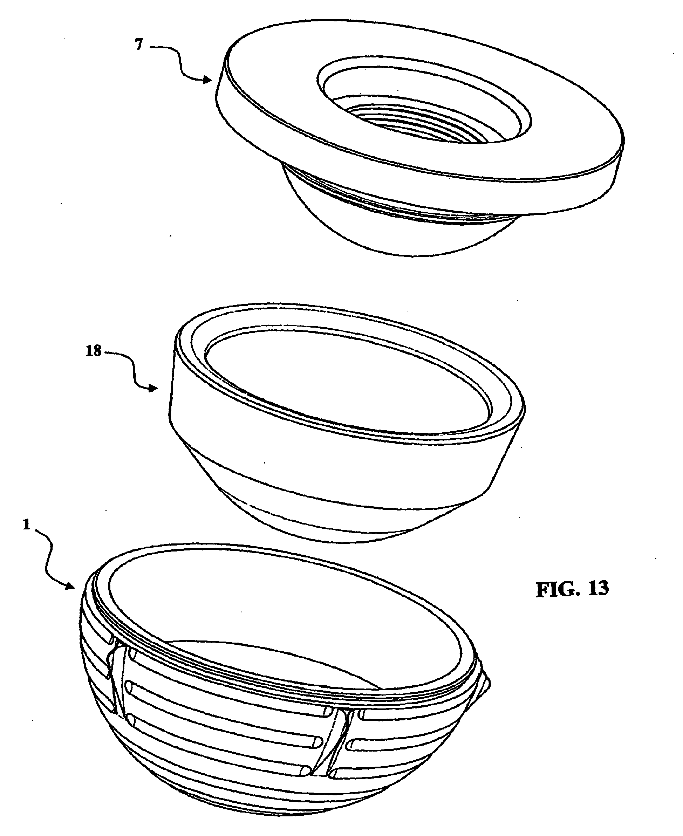

L'objet de la présente invention est illustré sur les figures 4 à 13. Sur ces figures, la structure annulaire externe de réception 6 et la structure périphérique annulaire de fixation 10 sont conformées de telle sorte que l'insert de pose et d'orientation 7 est engagé en force autour de la structure annulaire externe de réception 6 et est retenu en force par un serrage radial de la structure périphérique annulaire de fixation 10 sur la structure annulaire externe de réception 6.

Afin d'améliorer encore la tenue de l'insert de pose et d'orientation 7 sur la cupule d'insertion 1, on peut prévoir une coopération supplémentaire entre l'insert de pose et d'orientation 7 et la cupule d'insertion 1 au moyen d'une ou plusieurs nervures de verrouillage s'engageant dans une gorge de verrouillage.

Dans une première variante, illustrée sur les figures 4 à 7, on prévoit que :
- la face de réception 9 de la structure annulaire externe de réception 6 comporte une gorge de verrouillage 14 périphérique discontinue,
- la face de liaison 11 de la structure périphérique annulaire de fixation 10 de l'insert de pose et d'orientation 7 comporte une pluralité de nervures de verrouillage 15a à 15d réparties en périphérie et conformées pour s'engager dans la gorge de verrouillage 14 périphérique discontinue.

Dans une seconde variante, illustrée sur les figures 8 et 9, la face de réception 9 de la structure annulaire externe de réception 6 comporte une gorge de verrouillage 16 annulaire périphérique continue tandis que la face de liaison 11 de la structure périphérique annulaire de fixation 10 comporte une nervure de verrouillage 17 annulaire périphérique continue conformée pour s'engager dans la gorge de verrouillage 16 annulaire périphérique continue.

Dans cette deuxième variante, il est aussi possible que la face de liaison 11 de la structure périphérique annulaire de fixation 10 comporte une nervure de verrouillage annulaire périphérique discontinue. L'insert de pose et d'orientation 7 peut alors être semblable à celui de la première variante du deuxième mode de réalisation de l'invention, illustré sur les figures 6 et 7.

La figure 10 est une vue partielle en coupe du bord annulaire 4 de la cupule d'insertion 1 de la figure 8. Sur cette figure 10, on voit plus particulièrement que la structure annulaire externe de réception 6 comprend un décrochement radial périphérique d avec une face de réception 9 orientée vers l'extérieur. Le décrochement radial périphérique d a une épaisseur e1 comprise entre environ 0,7 mm et environ 1,2 mm.

La face de réception 9 comporte la gorge de verrouillage 16 annulaire périphérique continue. La gorge de verrouillage 16 annulaire périphérique continue présente une épaisseur radiale e2 comprise entre environ 0,2 mm et environ 0,6 mm.

La cupule d'insertion 1 comporte une épaisseur e4 au voisinage de son bord annulaire 4. L'épaisseur e4 peut être faible, inférieure à 3 mm par exemple.

Le décrochement radial périphérique d du bord annulaire 4 a une hauteur h1 comprise entre environ 1 mm et environ 4 mm.

Enfin, la gorge de verrouillage 16 annulaire périphérique continue présente une hauteur h2 comprise entre environ 0,4 mm et 3 mm.

Toutes les dimensions énoncées ci-dessus en lien avec la deuxième variante sont valables pour la première variante ainsi que pour l'objet des figures 1 à 3.

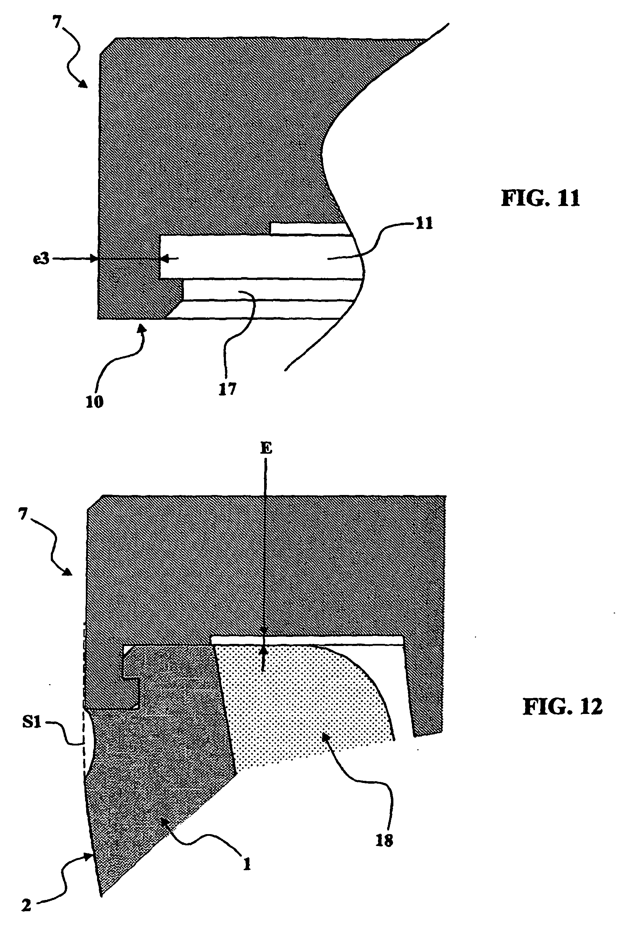

Comme on le voit sur la figure 11, la structure périphérique annulaire de fixation 10 de l'insert de pose et d'orientation 7 présente, avec sa face de liaison 11 et sa nervure de verrouillage 17 annulaire périphérique continue, une forme et des dimensions complémentaires de la face de réception 9 et de la gorge de verrouillage 16 annulaire périphérique continue. L'insert de pose et d'orientation 7 et la cupule d'insertion 1 peuvent être ainsi être associés de façon très intime, comme illustré sur la figure 12.

Afin d'éviter tout conflit de l'insert de pose et d'orientation 7 avec la masse osseuse autour de la cavité cotyloïdienne naturelle du bassin du patient, l'insert de pose et d'orientation 7 et la structure annulaire externe de réception 6 de la cupule d'insertion 1 sont conformés dé telle sorte que, lorsque l'insert de pose et d'orientation 7 est fixé à la structure annulaire externe de réception 6 de la cupule d'insertion 1, l'insert de pose et d'orientation 7 ne dépasse pas à l'extérieur d'une surface sensiblement hémisphérique S1 (illustrée en pointillés sur la figure 12) définie par la face extérieure d'ancrage 2 convexe sensiblement hémisphérique de la cupule d'insertion 1.

Pour ce faire, la structure périphérique annulaire de fixation 10 de l'insert de pose et d'orientation 7 présente une épaisseur radiale e3 sensiblement égale ou inférieure à l'épaisseur e1 du décrochement radial d du bord annulaire 4.

Afin de ne pas risquer d'endommager la cupule d'insertion 1, l'insert de pose et d'orientation 7 est avantageusement fabriqué en matière plastique, de préférence en polyéthylène qui est une matière peu onéreuse et facile à usiner.

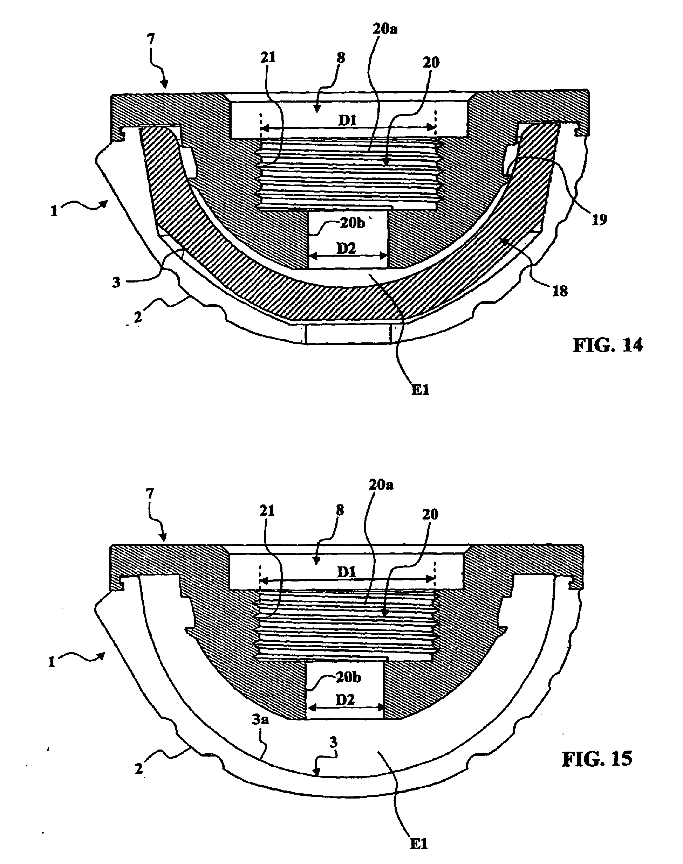

Dans le cadre de l'invention, la face de réception interne 3 concave peut être une face de glissement 3a pour la réception d'un insert articulaire mobile, pour un cotyle à double mobilité par exemple. Dans ce cas, la face de glissement 3a est destinée à recevoir un insert articulaire mobile sphérique (figure 15).

Toujours dans le cadre de l'invention, la face de réception interne 3 concave peut être conformée pour la réception d'un insert articulaire immobile 18, pour un cotyle à simple mobilité par exemple, comme illustré sur la figure 14. L'insert articulaire immobile 18 est immobilisé dans la cupule d'insertion 1 par un engagement conique.

On voit plus particulièrement sur la figure 14 qu'il est possible d'effectuer en usine un préassemblage et un conditionnement à l'état stérile de l'insert articulaire 18 engagé dans la cupule d'insertion 1 avec l'insert de pose et d'orientation 7 fixé à la cupule d'insertion 1. On dispose ainsi d'un ensemble préassemblé prêt à être impacté par le chirurgien, ce qui permet de réduire les temps opératoires. En outre, l'insert articulaire 18 est déjà installé en bonne place dans la cupule d'insertion 1, ce qui limite le risque d'un mauvais positionnement de celui-ci par le chirurgien. Ceci est particulièrement important lorsque l'insert articulaire 18 est en céramique car un mauvais positionnement de celui-ci conduit très souvent à une casse prématurée de la céramique.

Sur la figure 14, on voit plus particulièrement que l'insert de pose et d'orientation 7 comporte des moyens élastiques 19 de maintien de l'insert articulaire 18 en céramique dans la cupule d'insertion 1. Les moyens élastiques 19 permettent d'éviter tout déplacement de l'insert articulaire 18 dans la cupule d'insertion 1 lors du transport, du stockage et de la manutention de l'insert articulaire 18 et de la cupule d'insertion préassemblé.

Afin de ne pas risquer d'endommager l'insert articulaire 18 en céramique lors de l'impaction de la cupule d'insertion 1, lorsque l'insert de pose et d'orientation 7 est fixé à la cupule d'insertion 1, l'insert de pose et d'orientation 7 ne vient en contact avec l'insert articulaire 18 que selon les moyens élastiques 19 de maintien. On voit plus particulièrement sur la figure 12 que l'insert de pose et d'orientation 7 est conformé de telle sorte qu'il subsiste un espace E entre l'insert de pose et d'orientation 7 et l'insert articulaire 18. il n'est ainsi transmis aucun choc d'impaction à l'insert articulaire 18 en céramique qui est maintenu plaqué dans le fond de la cupule d'insertion 1 par les moyens élastiques 19.

On voit plus particulièrement sur les figures 14 et 15 que la structure d'assemblage 8 de l'insert de pose et d'orientation 7 comprend un trou de fixation 20 à taraudage interne 21. Le taraudage interne 21 permet le vissage d'une portion filetée correspondante de l'impacteur (non représenté).

Après l'impaction de la cupule d'insertion 1 dans la cavité cotyloïdienne du bassin du patient, il est nécessaire de procéder au retrait de l'insert de pose et d'orientation 7. Il est nécessaire que ce retrait s'effectue sans induire d'effort ou de contrainte entre la face extérieure d'ancrage 2 et la cavité cotyloïdienne du bassin du patient pour ne pas détruire la liaison réalisée entre celles-ci lors de l'impaction.

Une première solution de séparation consiste alors à prévoir que le trou de fixation 20 est un trou débouchant, apte à coopérer avec un outil de désolidarisation comportant une tige filetée à extrémité distale conformée pour prendre appui, directement ou indirectement contre la face de réception interne 3 concave de la cupule d'insertion 1 lors du vissage de la tige filetée dans le trou débouchant. Sur les figures 14 et 15, le trou de fixation 20 comporte un premier tronçon 20a de diamètre D1 et un second tronçon 20b de diamètre D2 plus petit. La tige filetée de l'outil de désolidarisation présente un diamètre extérieur adapté pour que celle-ci vienne se visser dans le tronçon 20b de diamètre D2 du trou de fixation 20 débouchant.

Une seconde solution de séparation consiste à prévoir que :
- l'insert de pose et d'orientation 7 est conformé de façon à ce qu'il reste un espace libre E1 entre l'insert de pose et d'orientation 7 et le fond de la face de réception interne 3 concave (figure 15), ou, le cas échéant, entre l'insert de pose et d'orientation 7 et l'insert articulaire 18 (figure 14), une fois l'insert de pose et d'orientation 7 fixé à la structure annulaire externe de réception 6 de la cupule d'insertion 1,
- l'insert de pose et d'orientation 7 est en contact de façon étanche selon sa face de liaison 11 contre la face de réception 9 de la structure annulaire externe de réception 6,
- le trou de fixation 20 est un trou débouchant, mettant en communication avec l'extérieur l'espace libre E1 et dimensionné pour y engager l'extrémité d'une seringue de façon étanche.

Dans le cas des figures 14 et 15, l'extrémité de seringue présente un diamètre extérieur adapté pour venir s'engager de façon étanche dans le tronçon de diamètre D2 du trou de fixation 20.

On injecte ainsi sous pression un liquide tel que de l'eau ou un sérum physiologique dans l'espace libre E1 pour induire une force de séparation entre la cupule d'insertion 1 et l'insert de pose et d'orientation 7.

Afin d'obtenir un contact étanche entre l'insert de pose et d'orientation 7 et la cupule d'insertion 1, on utilisera de préférence la cupule d'insertion 1 et l'insert de pose et d'orientation 7 illustrés sur les figures 8 et 9.

Dans le cadre de la présente invention, lors de l'assemblage de l'ensemble comprenant la cupule d'insertion 1 et un insert de pose et d'orientation 7 (et éventuellement un insert articulaire 18 immobile), il est important de ne pas dégrader la structure annulaire de réception 6 et la structure périphérique annulaire de fixation 10. Or, afin de réaliser un serrage radial de la structure périphérique annulaire de fixation 10 sur la structure annulaire externe de réception 6, il est nécessaire que la structure périphérique annulaire de fixation 10 présente des dimensions diamétrales égales ou inférieures à celles de la structure annulaire externe de réception 6.

On a alors recours à un procédé d'assemblage par dilatation puis retrait au cours duquel on chauffe l'insert de pose et d'orientation 7 afin d'en augmenter les dimensions, puis on engage la structure périphérique annulaire de fixation 10 autour de la face de réception 9 de la structure annulaire externe de réception 6, et on ramène enfin à température ambiante l'insert de pose et d'orientation 7 pour en diminuer les dimensions.

Un tel procédé permet un assemblage sans émousser ni endommager la ou les nervures de verrouillage 15a-15d ou 17 qui conservent ainsi des arêtes vives pour une liaison plus forte entre l'insert de pose et d'orientation 7 et la cupule d'insertion 1.

Ce procédé de fabrication par chauffage permet également d'éviter les inconvénients précités du procédé de fabrication par refroidissement décrit dans le document WO 2006/040483 A1.

Lors de son retour à température ambiante, il se produit un serrage radial de la structure périphérique annulaire de fixation 10 sur la structure annulaire externe de réception 6. Pour l'assemblage de l'ensemble illustré sur la figure 14, on peut insérer, préalablement à la fixation de l'insert de pose et d'orientation 7 sur la cupule d'insertion 1, un insert articulaire 18 dans la face de réception interne 3 concave de la cupule d'insertion 1.

On peut ensuite procéder à une étape de stérilisation de l'ensemble ainsi formé et emballé dans une enveloppe de protection microbienne. Une stérilisation satisfaisante sera obtenue par bombardement de rayons gamma, de préférence selon une dose comprise entre environ 25 kGy et environ 40 kGy.

Il est expressément souligné que le procédé de fabrication et d'assemblage des ensembles des figures 14 et 15 par chauffage de l'insert de pose et d'orientation 7 puis retour à température ambiante peut être utilisé avec une cupule d'insertion 1 dont la structure annulaire externe de réception 6 n'est pas obligatoirement située en retrait du plan d'ouverture P. Le procédé de fabrication pourra ainsi faire l'objet d'une protection indépendamment de la position de la structure annulaire externe de réception 6 par rapport au plan d'ouverture P de la cupule d'insertion 1.

La présente invention n'est pas limitée aux modes de réalisation qui ont été explicitement décrits, mais elle en inclut les diverses variantes et généralisations contenues dans le domaine des revendications ci-après.

## Revendications

1. Procédé de fabrication d'un cotyle prothétique de hanche, comprenant les étapes de :
a) prévoir une cupule d'insertion (1) ayant une face de réception interne (3) concave à face d'ouverture (5) comprise dans un plan d'ouverture (P), et ayant une structure annulaire de réception externe (6),
b) prévoir un insert de pose et d'orientation (7) à structure périphérique annulaire de fixation (10) conformée pour coopérer avec la face de réception (9) de la structure annulaire externe de réception (6) en s'engageant autour de la structure annulaire externe de réception (6),
c) chauffer l'insert de pose et d'orientation (7) afin d'en augmenter les dimensions,
d) engager la structure périphérique annulaire de fixation (10) autour de la face de réception (9) de la structure annulaire externe de réception (6),
e) ramener à température ambiante l'insert de pose et d'orientation (7) pour en diminuer les dimensions de façon à obtenir un serrage radial de la structure périphérique annulaire de fixation (10) sur la structure annulaire externe de réception (6).

2. Procédé selon la revendication 1, **caractérisé en ce qu'**il comprend en outre, avant fixation de l'insert de pose et d'orientation (7) sur la cupule d'insertion (1), l'étape supplémentaire b1) d'insérer un insert articulaire (18) dans la face de réception interne (3) concave de la cupule d'insertion (1).

3. Procédé selon la revendication 2, **caractérisé en ce que** :
- l'insert articulaire (18) est en céramique,
- l'insert de pose et d'orientation (7) comporte des moyens élastiques (19) de maintien de l'insert articulaire (18) dans la cupule d'insertion (1),
- lorsque l'insert de pose et d'orientation (7) est fixé à la cupule d'insertion (1), l'insert de pose et d'orientation (7) ne vient en contact avec l'insert articulaire (18) que selon les moyens élastiques (19) de maintien de l'insert articulaire (18) dans la cupule d'insertion (1).

4. Procédé selon l'une quelconque des revendications 1 à 3, **caractérisé en ce qu'**il comporte en outre une étape f) au cours de laquelle on stérilise l'ensemble ainsi formé et emballé dans une enveloppe de protection microbienne.

5. Procédé selon la revendication 4, **caractérisé en ce que** l'insert articulaire (18) est en céramique et **en ce que** l'étape f) de stérilisation est réalisée par bombardement de rayons gamma, de préférence selon une dose comprise entre environ 25 kGy et environ 40 kGy.

6. Procédé selon l'une quelconque des revendications 1 à 5, **caractérisé en ce que** l'insert de pose et d'orientation (7) est en polyéthylène.

7. Procédé selon l'une quelconque des revendications 1 à 6, **caractérisé en ce que** :
- la structure annulaire externe de réception (6) comprend un décrochement radial périphérique (d) continu ou discontinu du bord annulaire (4), avec une face de réception (9) orientée vers l'extérieur,
- l'insert de pose et d'orientation (7) comporte une structure périphérique annulaire de fixation (10) continue ou discontinue à face de liaison (11) orientée vers l'intérieur, conformée pour coopérer avec la face de réception (9) de la structure annulaire externe de réception (6).

8. Procédé selon la revendication 7, **caractérisé en ce que** :
- la face de réception (9) de la structure annulaire externe de réception (6) comporte une gorge de verrouillage (14) périphérique discontinue,
- la face de liaison (11) de la structure périphérique annulaire de fixation (10) de l'insert de pose et d'orientation (7) comporte une pluralité de nervures de verrouillage (15a-15d) réparties en périphérie et conformées pour s'engager dans la gorge de verrouillage (14) périphérique discontinue.

9. Procédé selon la revendication 7, **caractérisé en ce que** :
- l'une de la face de réception (9) de la structure annulaire externe de réception (6) ou de la face de liaison (11) de la structure périphérique annulaire de fixation (10) comporte une gorge de verrouillage (16) annulaire périphérique continue,
- l'autre de la face de liaison (11) de la structure périphérique annulaire de fixation (10) ou de la face de réception (9) de la structure annulaire externe de réception (6) comporte une nervure de verrouillage (17) annulaire périphérique continue ou discontinue conformée pour s'engager dans la gorge de verrouillage (16) annulaire périphérique continue.

10. Procédé selon l'une quelconque des revendications 7 à 9, **caractérisé en ce que** :
- le décrochement radial périphérique (d) du bord annulaire (4) a une épaisseur (e1) comprise entre environ 0,7 mm et environ 1,2 mm,
- ladite gorge de verrouillage (14) périphérique discontinue ou ladite gorge de verrouillage (16) annulaire périphérique continue de la structure annulaire externe de réception (6) a une épaisseur radiale (e2) comprise entre environ 0,2 mm et environ 0,6 mm.

11. Procédé selon l'une quelconque des revendications 7 à 10, **caractérisé en ce que** le décrochement radial périphérique (d) du bord annulaire (4) a une hauteur (h1) comprise entre environ 1 mm et environ 4 mm.

12. Procédé selon l'une des revendications 8 ou 9, **caractérisé en ce que** ladite gorge de verrouillage (14) périphérique discontinue ou ladite gorge de verrouillage (16) annulaire périphérique continue de la structure annulaire externe de réception (6) a une hauteur (h2) comprise entre environ 0,4 mm et environ 3 mm.

13. Procédé selon l'une des revendication 7 à 12, **caractérisé en ce que** l'insert de pose et d'orientation (7) et la structure annulaire externe de réception (6) de la cupule d'insertion (1) sont conformés de telle sorte que, lorsque l'insert de pose et d'orientation (7) est fixé à la structure annulaire externe de réception (6) de la cupule d'insertion (1), l'insert de pose et d'orientation (7) ne dépasse pas à l'extérieur d'une-surface sensiblement hémisphérique (S1) définie par la face extérieure d'ancrage (2) convexe sensiblement hémisphérique de la cupule d'insertion (1).

14. Procédé selon la revendication 13, **caractérisé en ce que** la structure périphérique annulaire de fixation (10) de l'insert de pose et d'orientation (7) présente une épaisseur (e3) radiale sensiblement égale ou inférieure à l'épaisseur (e1) du décrochement radial (d) du bord annulaire (4).

15. Procédé selon l'une quelconque des revendications 1 à 14, **caractérisé en ce que** la structure annulaire de réception (6) est externe et située en retrait du plan d'ouverture (P).

16. Procédé selon l'une quelconque des revendications 1 à 15, **caractérisé en ce que** l'insert de pose et d'orientation (7) comporte une structure d'assemblage (8) sur laquelle un impacteur peut être fixé de façon amovible.

17. Procédé selon la revendication 16, **caractérisé en ce que** la structure d'assemblage (8) comprend un trou de fixation (20) à taraudage interne (21) pratiqué dans l'insert de pose et d'orientation (7), permettant le vissage d'une portion filetée correspondante de l'impacteur.

18. Procédé selon la revendication 17, **caractérisé en ce que** le trou de fixation (20) est un trou débouchant, apte à coopérer avec un outil de désolidarisation comportant une tige filetée apte à se visser dans le trou débouchant et qui a une extrémité distale conformée pour prendre appui, directement ou indirectement, contre la face de réception (3) interne concave de la cupule d'insertion (1) lors du vissage de la tige filetée dans le trou débouchant.

19. Procédé selon la revendication 17, **caractérisé en ce que** :
- l'insert de pose et d'orientation (7) est conformé de façon à ce qu'il reste un espace libre (E1) entre l'insert de pose et d'orientation (7) et le fond de la face de réception interne (3) concave, ou, le cas échéant, entre l'insert de pose et d'orientation (7) et l'insert articulaire (18), une fois l'insert de pose et d'orientation (7) fixé à la structure annulaire externe de réception (6) de la cupule d'insertion (1),
- l'insert de pose et d'orientation (7) est en contact de façon étanche selon sa face de liaison (11) contre la face de réception (9) de la structure annulaire externe de réception (6),
- le trou de fixation (20) est un trou débouchant, mettant en communication avec l'extérieur l'espace libre (E1) entre l'insert de pose et d'orientation (7) et le fond de la face de réception interne (3) concave, ou, le cas échéant, entre l'insert de pose et d'orientation (7) et l'insert articulaire (18), et dimensionné pour y engager l'extrémité d'une seringue de façon étanche.

20. Procédé selon l'une quelconque des revendications 1 à 19, **caractérisé en ce que** la cupule d'insertion (1) est métallique.

21. Procédé selon l'une quelconque des revendications 1 à 19, **caractérisé en ce que** la cupule d'insertion (1) est en PEEK.

## Patentansprüche

1. Verfahren zum Herstellen einer Hüftgelenkpfannenprothese, umfassend die Schritte:
a) Bereitstellen eines Pfanneneinsatzes (1), der eine konkave innere Aufnahmefläche (3) mit einer in einer Öffnungsebene (P) enthaltenen Öffnungsfläche (5) aufweist, und eine äußere ringförmige Aufnahmestruktur (6) aufweist,
b) Bereitstellen eines Installations- und Ausrichtungseinsatzes (7), der eine ringförmige Befestigungsstruktur (10) aufweist, die dazu vorgesehen ist, mit der Aufnahmefläche (9) der äußeren ringförmigen Aufnahmestruktur (6) durch Verkuppeln um die äußere ringförmige Aufnahmestruktur (6) zusammenzuwirken,
c) Erhitzen des Installations- und Ausrichtungseinsatzes (7), um seine geometrische Ausdehnung zu erhöhen,
d) Verkuppeln der ringförmigen Befestigungsstruktur (10) um die Aufnahmefläche (9) der äußeren ringförmigen Aufnahmestruktur (6),
e) Rückführen des Installations- und Ausrichtungseinsatzes (7) auf Umgebungstemperatur um seine geometrische Ausdehnung zu reduzieren, so dass ein radiales Verspannen der ringförmigen Befestigungsstruktur (10) auf der äußeren ringförmigen Aufnahmestruktur (6) erreicht wird.

2. Verfahren nach Anspruch 1, **dadurch gekennzeichnet, dass** das Verfahren vor dem Fixieren des Installations- und Ausrichtungseinsatzes (7) am Pfanneneinsatz (1) zusätzlich den ergänzenden Schritt b1) des Einsetzens eines Gelenkeinsatzes (18) in die konkave innere Aufnahmefläche (3) des Pfanneneinsatzes (1) umfasst.

3. Verfahren nach Anspruch 2, **dadurch gekennzeichnet, dass**:
- der Gelenkeinsatz (18) aus Keramik gefertigt ist,
- der Installations- und Ausrichtungseinsatz (7) elastische Mittel (19) zum Halten des Gelenkeinsatzes (18) im Pfanneneinsatz (1) aufweist,
- wenn der Installations- und Ausrichtungseinsatz (7) am Pfanneneinsatz (1) fixiert ist, der Installations- und Ausrichtungseinsatz (7) nur über die elastischen Mittel (19) zum Halten des Gelenkeinsatzes (18) im Pfanneneinsatz (1) mit dem Gelenkeinsatz (18) in Kontakt kommt.

4. Verfahren nach einem der Ansprüche 1 bis 3, **dadurch gekennzeichnet, dass** das es einen zusätzlichen Schritt f) aufweist, während dessen Ausführung man die so gebildete und in einer mikrobiologischen Schutzhülle gepackte Einheit sterilisiert.

5. Verfahren nach Anspruch 4, **dadurch gekennzeichnet, dass** der Gelenkeinsatz (18) aus Keramik ist und wobei der Sterilisationsschritt f) durch Bestrahlen mit Gammastrahlen vorzugsweise bei einer Dosis zwischen ungefähr 25 kGy und ungefähr 40 kGy durchgeführt wird.

6. Verfahren nach einem der Ansprüche 1 bis 5, **dadurch gekennzeichnet, dass** der Installations- und Ausrichtungseinsatz (7) aus Polyethylen gefertigt ist.

7. Verfahren nach einem der Ansprüche 1 bis 6, **dadurch gekennzeichnet, dass**:
- die äußere ringförmige Aufnahmestruktur (6) eine aus der ringförmigen Kante (4) gebildete durchgehende oder unterbrochene radiale Schulter (d) mit einer Aufnahmefläche (9) umfasst, die zur Außenseite gerichtet ist,
- der Installations- und Ausrichtungseinsatz (7) eine durchgehende oder unterbrochene ringförmige Befestigungsstruktur (10) mit einer Verbindungsfläche (11) aufweist, die zur Innenseite gerichtet und vorgesehen ist, mit der Aufnahmefläche (9) der äußeren ringförmigen Aufnahmestruktur (6) zusammenzuwirken.

8. Verfahren nach Anspruch 7, **dadurch gekennzeichnet, dass**:
- die Aufnahmefläche (9) der äußeren ringförmigen Aufnahmestruktur (6) eine unterbrochene umfänglich verlaufende Verriegelungskehle (14) aufweist,
- die Verbindungsfläche (11) der umfänglich verlaufenden ringförmigen Befestigungsstruktur (10) des Installations- und Ausrichtungseinsatzes (7) eine Vielzahl von Verriegelungsrippen (15a-15d) aufweist, die umfänglich verteilt und vorgesehen sind, in die umfänglich verlaufende Verriegelungskehle (14) einzugreifen.

9. Verfahren nach Anspruch 7, **dadurch gekennzeichnet, dass**:
- entweder die Aufnahmefläche (9) der äußeren ringförmigen Aufnahmestruktur (6) oder die Verbindungsfläche (11) der umfänglich verlaufenden ringförmigen Befestigungsstruktur (10) eine durchgehende umfänglich verlaufende Verriegelungskehle (16) aufweist,
- die andere der Verbindungsfläche (11) der umfänglich verlaufenden ringförmigen Befestigungsstruktur (10) oder der Aufnahmefläche (9) der äußeren ringförmigen Aufnahmestruktur (6) eine durchgehende oder unterbrochene umfänglich verlaufende ringförmige Verriegelungsrippe (17) aufweist, die vorgesehen ist in die durchgehende umfänglich verlaufende Verriegelungskehle (16) einzugreifen.

10. Verfahren nach einem der Ansprüche 7 bis 9, **dadurch gekennzeichnet, dass**:
- die umfänglich verlaufende radiale Schulter (d) der ringförmigen Kante (4) eine Dicke (e1) zwischen ungefähr 0,7 mm und ungefähr 1,2 mm aufweist,
- die unterbrochene umfänglich verlaufende Verriegelungskehle (14) oder die durchgehende umfänglich verlaufende ringförmige Verriegelungskehle (16) der äußeren ringförmigen Aufnahmestruktur (6) eine radiale Dicke (e2) zwischen ungefähr 0,2 mm und ungefähr 0,6 mm aufweist.

11. Verfahren nach einem der Ansprüche 7 bis 10, **dadurch gekennzeichnet, dass** die umfänglich verlaufende radiale Schulter (d) der ringförmigen Kante (4) eine Höhe (h1) zwischen ungefähr 1 mm und ungefähr 4 mm aufweist.

12. Verfahren nach einem der Ansprüche 8 oder 9, **dadurch gekennzeichnet, dass** die unterbrochene umfänglich verlaufende Verriegelungskehle (14) oder die durchgehend umfänglich verlaufende ringförmige Verriegelungskehle (16) der äußeren ringförmigen Aufnahmestruktur (6) eine Höhe (h2) zwischen ungefähr 0,4 mm und ungefähr 3 mm aufweist.

13. Verfahren nach einem der Ansprüche 7 bis 12, **dadurch gekennzeichnet, dass** der Installations- und Ausrichtungseinsatz (7) und die äußere ringförmige Aufnahmestruktur (6) des Pfanneneinsatzes (1) derart ausgelegt sind, dass wenn der Installations- und Ausrichtungseinsatz (7) an äußeren ringförmigen Aufnahmestruktur (6) des Pfanneneinsatzes (1) befestigt ist, der Installationsund Ausrichtungseinsatz (7) nicht vor eine halbkugelförmige Oberfläche (S1) ragt, die durch die im Wesentlichen halbkugelförmige ausgebuchtete äußere Verankerungsfläche (2) des Pfanneneinsatzes (1) vorgegeben ist.

14. Verfahren nach Anspruch 13, **dadurch gekennzeichnet, dass** die umfänglich verlaufende ringförmige Befestigungsstruktur (10) des Installations- und Ausrichtungseinsatzes (7) eine radiale Dicke (e3) aufweist, die im Wesentlichen gleich oder kleiner als die Dicke (e1) der radialen Schulter (d) der ringförmigen Ecke (4) ist.

15. Verfahren nach einem der Ansprüche 1 bis 14, **dadurch gekennzeichnet, dass** die ringförmige Aufnahmestruktur (6) außen liegend und zurückgesetzt von der Öffnungsebene (P) ausgebildet ist.

16. Verfahren nach einem der Ansprüche 1 bis 15, **dadurch gekennzeichnet, dass** der Installations- und Ausrichtungseinsatz (7) eine Fügestruktur (8) umfasst auf der ein Impaktor lösbar fixiert werden kann.

17. Verfahren nach Anspruch 16, **dadurch gekennzeichnet, dass** die Fügestruktur (8) eine im Installations- und Ausrichtungseinsatz (7) ausgebildete Befestigungsbohrung (20) mit einem Innengewinde (21) umfasst, die ein Verschrauben eines entsprechenden Gewindebereiches des Impaktors erlaubt.

18. Verfahren nach Anspruch 17, **dadurch gekennzeichnet, dass** die Befestigungsbohrung (20) eine Durchgangsbohrung ist, die mit einem Lösewerkzeug zusammenwirken kann, das einen Gewindestab umfasst, der in die Durchgangsbohrung eingeschraubt werden kann und der ein äußeres Ende aufweist, das eingerichtet ist, unmittelbar oder mittelbar gegen die konkave innere Aufnahmefläche (3) des Pfanneneinsatzes (1) gestützt zu werden, wenn der Gewindestab in die Durchgangsbohrung eingeschraubt ist.

19. Verfahren nach Anspruch 17, **dadurch gekennzeichnet, dass**:
- der Installations- und Ausrichtungseinsatz (7) der ausgebildet ist, dass ein freier Raum (E1) zwischen dem Installations- und Ausrichtungseinsatz (7) und dem Boden der konkaven inneren Aufnahmefläche (3), oder, falls geeignet, zwischen dem Installations- und Ausrichtungseinsatz (7) und dem Gelenkeinsatz (18) verbleibt, sobald der Installations- und Ausrichtungseinsatz (7) an der äußeren ringförmigen Aufnahmestruktur (6) des Pfanneneinsatzes (1) fixiert ist,
- der Installations- und Ausrichtungseinsatz (7) in versiegelter Weise über seine Anschlussfläche (11) gegen die Aufnahmefläche (9) der äußeren ringförmigen Aufnahmestruktur (6) gestützt ist,
- die Befestigungsbohrung (20) eine Durchgangsbohrung ist, über die der freie Raum (E1) zwischen dem Installations- und Ausrichtungseinsatz (7) und dem Boden der konkaven inneren Aufnahmefläche (3), oder, falls geeignet, zwischen dem Installationsund Ausrichtungseinsatz (7) und dem Gelenkeinsatz (18) mit der Außenseite verbunden wird, und die für einen austrittsdichten Eingriff des Endes einer Spritze eingerichtet ist.

20. Verfahren nach einem der vorstehenden Ansprüche 1 bis 19, wobei der Pfanneneinsatz (1) metallisch ist.

21. Verfahren nach einem der vorstehenden Ansprüche 1 bis 19, wobei der Pfanneneinsatz (1) ein PEEK ist.

## Claims

1. Method for manufacturing a prosthetic hip acetabulum, comprising the steps of:
a) providing an insertion cup (1) having a concave inner receiving face (3) with an opening face (5) contained in an opening plane (P), and having an outer annular receiving structure (6),
b) providing an installation and orientation insert (7) having a peripheral annular fixing structure (10) designed to cooperate with the receiving face (9) of the outer annular receiving structure (6) by engaging around the outer annular receiving structure (6),
c) heating the installation and orientation insert (7) in order to increase the dimensions thereof,
d) engaging the peripheral annular fixing structure (10) around the receiving face (9) of the outer annular receiving structure (6),
e) bringing the installation and orientation insert (7) back to room temperature in order to reduce the dimensions thereof, so as to achieve a radial clamping of the peripheral annular fixing structure (10) on the outer annular receiving structure (6).

2. Method as claimed in claim 1, **characterized in that**, before fixing the installation and orientation insert (7) on the insertion cup (1), the method additionally comprises the supplementary step b1) of inserting an articular insert (18) in the concave inner receiving face (3) of the insertion cup (1).

3. Method as claimed in claim 2, **characterized in that**:
- the articular insert (18) is made of ceramic,
- the installation and orientation insert (7) has elastic means (19) for holding the articular insert (18) in the insertion cup (1),
- when the installation and orientation insert (7) is fixed to the insertion cup (1), the installation and orientation insert (7) comes into contact with the articular insert (18) only via the elastic means (19) for holding the articular insert (18) in the insertion cup (1).

4. Method as claimed in any one of claims 1 through 3, **characterized in that** it additionally has a step f) during which the unit thus formed and packed in a microbial protection envelope is sterilized.

5. Method as claimed in claim 4, **characterized in that** the articular insert (18) is made of ceramic, and **in that** the step f) of sterilization is carried out by bombardment with gamma rays, preferably at a dose of between approximately 25 kGy and approximately 40 kGy.

6. Method as claimed in any one of claims 1 through 5, **characterized in that** the installation and orientation insert (7) is made of polyethylene.

7. Method as claimed in any one of claims 1 through 6, **characterized in that**:
- the outer annular receiving structure (6) comprises a continuous or interrupted peripheral radial shoulder (d) of the annular edge (4), with a receiving face (9) directed toward the outside,
- the installation and orientation insert (7) has a continuous or interrupted peripheral annular fixing structure (10) with a connecting face (11) directed toward the inside and designed to cooperate with the receiving face (9) of the outer annular receiving structure (6).

8. Method as claimed in claim 7, **characterized in that**:
- the receiving face (9) of the outer annular receiving structure (6) has an interrupted peripheral locking groove (14),
- the connecting face (11) of the peripheral annular fixing structure (10) of the installation and orientation insert (7) has a plurality of locking ribs (15a-15d) which are distributed peripherally and are designed so as to engage in the interrupted peripheral locking groove (14).

9. Method as claimed in claim 7, **characterized in that**:
- either the receiving face (9) of the outer annular receiving structure (6) or the connecting face (11) of the peripheral annular fixing structure (10) has a continuous peripheral annular locking groove (16),
- the other of the connecting face (11) of the peripheral annular fixing structure (10) or the receiving face (9) of the outer annular receiving structure (6) has a continuous or interrupted peripheral annular locking rib (17) designed so as to engage in the continuous peripheral annular locking groove (16).

10. Method as claimed in any one of claims 7 through 9, **characterized in that**:
- the peripheral radial shoulder (d) of the annular edge (4) has a thickness (e1) of between approximately 0.7 mm and approximately 1.2 mm,
- said interrupted peripheral locking groove (14) or said continuous peripheral annular locking groove (16) of the outer annular receiving structure (6) has a radial thickness (e2) of between approximately 0.2 mm and approximately 0.6 mm.

11. Method as claimed in any one of claims 7 through 10, **characterized in that** the peripheral radial shoulder (d) of the annular edge (4) has a height (h1) of between approximately 1 mm and approximately 4 mm.

12. Method as claimed in either of claims 8 and 9, **characterized in that** said interrupted peripheral locking groove (14) or said continuous peripheral annular locking groove (16) of the outer annular receiving structure (6) has a height (h2) of between approximately 0.4 mm and approximately 3 mm.

13. Method as claimed in one of claims 7 through 12, **characterized in that** the installation and orientation insert (7) and the outer annular receiving structure (6) of the insertion cup (1) are designed in such a way that, when the installation and orientation insert (7) is fixed to the outer annular receiving structure (6) of the insertion cup (1), the installation and orientation insert (7) does not protrude outside a substantially hemispherical surface (S1) defined by the substantially hemispherical convex outer anchoring face (2) of the insertion cup (1).

14. Method as claimed in claim 13, **characterized in that** the peripheral annular fixing structure (10) of the installation and orientation insert (7) has a radial thickness (e3) substantially equal to or less than the thickness (e1) of the radial shoulder (d) of the annular edge (4).

15. Method as claimed in any one of claims 1 through 14, **characterized in that** the annular receiving structure (6) is external and is situated set back from the opening plane (P).

16. Method as claimed in any one of claims 1 through 15, **characterized in that** the installation and orientation insert (7) has an assembly structure (8) on which an impactor can be fixed removably.

17. Method as claimed in claim 16, **characterized in that** the assembly structure (8) comprises a fixation hole (20) with internal thread (21) made in the installation and orientation insert (7), permitting the screwing of a corresponding threaded portion of the impactor.

18. Method as claimed in claim 17, **characterized in that** the fixation hole (20) is a through-hole able to cooperate with a disconnecting tool having a threaded rod which is able to be screwed into the through-hole and which has a distal end designed to bear directly or indirectly against the concave inner receiving face (3) of the insertion cup (1) when the threaded rod is screwed into the through-hole.

19. Method as claimed in claim 17, **characterized in that**:
- the installation and orientation insert (7) is designed in such a way that a free space (E1) remains between the installation and orientation insert (7) and the bottom of the concave inner receiving face (3), or, if appropriate, between the installation and orientation insert (7) and the articular insert (18), once the installation and orientation insert (7) is fixed to the outer annular receiving structure (6) of the insertion cup (1),
- the installation and orientation insert (7) bears in a sealed manner across its connecting face (11) against the receiving face (9) of the outer annular receiving structure (6),
- the fixation hole (20) is a through-hole via which the free space (E1) between the installation and orientation insert (7) and the bottom of the concave inner receiving face (3) or, if appropriate, between the installation and orientation insert (7) and the articular insert (18) is brought into communication with the outside and which is dimensioned for leaktight engagement of the end of a syringe.

20. Method as claimed in any one of claims 1 through 19, **characterized in that** the insertion cup (1) is made of metal.

21. Method as claimed in any one of claims 1 through 19, **characterized in that** the insertion cup (1) is made of PEEK.
